# EUROPEAN PATENT APPLICATION

(11) **EP 3 045 503 A1**
(43) Date of publication of application: **20.07.2016**
(21) Application number: 15151341.3
(22) Date of filing: 15.01.2015
(51) Int. Cl.: C09C 1/02, A61K 8/19, C11D 3/14

(54) **Surface-treated calcium carbonate with improved stability in environments with a pH of 4.5 to 7**

(71) Applicant: Omya International AG, 4665 Oftringen (CH)
(72) Inventor: Gerard, Daniel E., 4058 Basel (CH); Budde, Tanja, 4800 Zofingen (CH); Gane, Patrick A. C., 4852 Rothrist (CH)
(74) Representative: Maiwald Patentanwalts GmbH

(57) **Abstract**

The present invention relates to a process for producing a surface-treated calcium carbonate with improved stability in environments with a pH of 4.5 to 7, wherein calcium carbonate, at least one acid having a pKₐ value from 0 to 8, when measured at 20°C, and at least one conjugate base are brought into contact to form surface-treated calcium carbonate.

## Description

The present invention relates to a surface-treated calcium carbonate, dry or in a suspension, with improved stability in environments with a pH of 4.5 to 7, a process for producing the same, and its use, as well as products containing a calcium carbonate with improved stability in environments with a pH of 4.5 to 7.

Abrasive cleaners are well-known in the art and are widely used for cleaning all kinds of surfaces, especially surfaces which become soiled with difficult to remove stains. For example, abrasive cleaners may be used for cleaning household surfaces such as tables, sinks or dish surfaces. Abrasive materials can also be used in personal care products such as toothpastes, as exfoliants in facial and body scrubs, or soaps. Besides natural ingredients such as crushed apricot kernel, almond shells, sugar or salt crystals, or pumice, polymer beads such as polyethylene microspheres are widely used in personal care products as abrasive materials.

The use of polyethylene microspheres, also called microbeads, however, in cosmetic compositions for abrasives and exfoliation has become a substantial environmental concern due to the water pollution by microplastics such as microbeads. Microbeads from cosmetics and personal care products can be washed down the drain after use, entering the sewerage system before making their way into rivers and canals. From there, they can easily end up in seas and oceans.

As a consequence, microbeads have already been banned in some states of the United States and in the Netherlands. Illinois became the first U.S. state to enact legislation banning the manufacture and sale of products containing microbeads: the two-part ban goes into effect in 2018 and 2019. The New York State Assembly voted in May 2014 to ban microbeads, and additional legislation is under consideration in Ohio and California. The Netherlands is the first country in the world to announce its intent to be virtually free of microbeads in cosmetics by the end of 2016. As a result, cosmetic companies have pledged to phase out the use of microbeads over the next years and search intensively for environmentally friendly substitutes.

A liquid cleaning composition comprising calcium carbonate as abrasive material is, for example, described in WO 2013/078949. A dry blasting process for the cleaning of solid surfaces involving the use of natural alkaline earth carbonate particles is disclosed in WO 2009/133173. WO 03/061908, EP 1 666 203, and EP 1 738 872 also disclose a process for dry cleaning of solid surfaces involving the use of a precipitate or agglomerate of an insoluble alkali metal carbonate. US 5,827,114, US 5,531,634, and WO 94/07658 disclose the use of calcium carbonate for blast cleaning. A microdermabrasion procedure is described in US 2001/0023351. The rounded particles used in this procedure can be glass beads.

However, conventional calcium carbonate decomposes in acidic aqueous media, and thus, generally cannot be used in compositions having a pH of less than 7, which is often the case for cleaning compositions or personal care products. Cosmetic abrasive cleaning compositions such as body scrubs, for example, typically have a pH value of about 5 to 7 in order to preserve the protective layer of the skin.

In view of the foregoing, there is a continuous need for environmentally friendly abrasive materials.

The use of acid stabilized calcium carbonates or suspensions thereof is known in the paper making industry for the production of neutral to acidic acid. Such uses are, for example, described in US 6,228,161, and US 7,033,428 (both of Drummond), US 5,043,017, US 5,156,719 (both of Passaretti), WO 97/08247, WO 97/08248, and WO 97/14651 (all ofECC), as well as in US 6,540,878 (Leino).

The reaction of ground calcium carbonate or precipitated calcium carbonate in the presence of at least one acid and carbon dioxide is described in WO 00/39222, WO 2004/083316, WO 2005/121257, WO 2009/074492, EP 2 264 108, EP 2 264 109, US 2004/0020410, and EP 2 070 991. All products obtained by the reactions described in these documents are porous products showing a high BET surface area in the range of 20 to 120 or to even 200 m²/g.

Accordingly it is an object of the present invention to provide an abrasive material, which avoids at least some of the foregoing disadvantages. In particular, it is desirable to provide an abrasive agent which is stable in environments with a pH of 4.5 to 7, and, therefore, can also be used in abrasive compositions that are stable under these conditions. Furthermore, it is desirable to provide an abrasive material which has gentle abrasive properties, and, therefore, can also be used for cleaning delicate surfaces and in personal care products.

Furthermore, it is an object of the present invention to provide an abrasive material which is derivable from natural sources. It is also desirable to provide an abrasive agent which is easily biodegradable.

The foregoing and other objects are solved by the subject-matter as defined herein in the independent claims.

According to one aspect of the present invention, a process for producing a surface-treated calcium carbonate with improved stability in environments with a pH of 4.5 to 7 is provided, wherein the process comprises the steps of:
i) providing calcium carbonate,
ii) providing at least one acid having a pKₐ value from 0 to 8, when measured at 20°C,
iii) providing at least one conjugate base,
iv) contacting the calcium carbonate of step i), the at least one acid of step ii), and the at least one conjugate base of step iii) to form surface-treated calcium carbonate,
wherein the at least one acid of step ii) and/or the at least one conjugate base of step iii) are provided in form of an aqueous solution.

According to a further aspect, a suspension of a surface-treated calcium carbonate with improved stability in environments with a pH of 4.5 to 7, and obtainable by a process according to the present invention is provided.

According to yet a further aspect, a dried surface-treated calcium carbonate with improved stability in environments with a pH of 4.5 to 7, and obtainable by a process according to the present invention is provided.

According to still a further aspect an abrasive cleaning composition comprising a suspension of a surface-treated calcium carbonate or a dried surface-treated calcium carbonate according to the present invention is provided.

According to still a further aspect, use of a suspension of a surface-treated calcium carbonate according to the present invention as abrasive material is provided, preferably as abrasive material for cleaning application.

According to yet a further aspect, use of a dried surface-treated calcium carbonate according to the present invention as abrasive material is provided, preferably as abrasive material for cosmetic application and/or cleaning application, more preferably as abrasive material for cosmetic application, and most preferably as abrasive material for topical skin application.

According to still a further aspect, use of an abrasive cleaning composition according to the present invention for cleaning a surface is provided, preferably for cleaning an animate surface, and more preferably for cleaning an animate surface selected from the group consisting of human skin, animal skin, human hair, animal hair, and tissues of the oral cavity such as teeth, gums, tongue or buccal surfaces.

Advantageous embodiments of the present invention are defined in the corresponding sub-claims.

According to one embodiment step iv) comprises the steps of: (a1) contacting the calcium carbonate of step i) and the at least one acid of step ii) to form a pre-treated calcium carbonate, and (a2) contacting the pre-treated calcium carbonate of step a1) with the at least one conjugate base of step iii) to form surface-treated calcium carbonate, wherein at least the at least one acid of step ii) is provided in form of an aqueous solution.

According to another embodiment step iv) comprises the steps of: (b1) contacting the calcium carbonate of step i) and the at least one conjugate base of step iii) to form a pre-treated calcium carbonate, and (b2) contacting the pre-treated calcium carbonate of step b1) with the at least one acid of step ii) to form surface-treated calcium carbonate, wherein at least the at least one conjugate base of step iii) is provided in form of an aqueous solution.

According to one embodiment the at least one acid of step ii) and the at least one conjugate base of step iii) are provided together in form of an aqueous buffer solution, and in step iv) the calcium carbonate of step i) is contacted with the aqueous buffer solution to form surface-treated calcium carbonate. According to another embodiment step iv) comprises the steps of: (c1) mixing the calcium carbonate of step i), the aqueous buffer solution, and optionally water, to form an aqueous suspension of surface-treated calcium carbonate, and (c2) separating the surface-treated calcium carbonate from the aqueous suspension obtained from step c1).

According to one embodiment the calcium carbonate of step i) is natural ground calcium carbonate, precipitated calcium carbonate, dolomite, agglomerates or aggregates thereof, or a mixture of the aforementioned materials, and preferably natural ground calcium carbonate. According to another embodiment the calcium carbonate of step i) is in form of particles having a volume determined median particle size *d*₅₀ from 1 to 1500 µm, preferably from 25 to 1 400 µm, more preferably from 100 from 1 200 µm, even more preferably from 200 to 1 000 µm, and most preferably from 300 to 800 µm

According to one embodiment the at least one acid of step ii) is selected from the group consisting of phosphoric acid, citric acid, sulphurous acid, boric acid, acetic acid, tartaric acid, formic acid, propanoic acid, oxalic acid, phosphonic acid, acidic salt, and mixtures thereof. According to a preferred embodiment the at least one acid of step ii) is selected from the group consisting of phosphoric acid, citric acid, tartaric acid, oxalic acid, their acidic salts, and mixtures thereof. According to the most preferred embodiment the at least one acid of step ii) is selected from the group consisting of phosphoric acid, citric acid, their acidic salts, and mixtures thereof.

According to another embodiment the at least one conjugate base of step iii) is an alkali metal salt and/or alkaline earth metal salt of an acid having a pKₐ value from 0 to 8, when measured at 20°C, preferably the at least one conjugate base of step iii) is an alkali metal salt and/or alkaline earth metal salt of an acid selected from the group consisting of phosphoric acid, citric acid, sulphurous acid, boric acid, acetic acid, tartaric acid, formic acid, propanoic acid, oxalic acid, phosphonic acid, and mixtures thereof, more preferably the at least one conjugate base of step iii) is a sodium and/or potassium salt of an acid selected from the group consisting of phosphoric acid, citric acid, sulphurous acid, boric acid, acetic acid, tartaric acid, formic acid, propanoic acid, oxalic acid, phosphonic acid, and mixtures thereof, and most preferably the at least one conjugate base of step iii) is a sodium and/or potassium salt of an acid selected from the group consisting of phosphoric acid, citric acid, and mixtures thereof. The alkali metal of the above alkali metal salt can be selected from the group consisting of Li⁺, Na⁺, K⁺, and is preferably selected from the group consisting of Na⁺ and K⁺. The metal of the above alkaline earth metal salt is Mg²⁺.

According to one embodiment the aqueous buffer solution has a pH value from 4.0 to 7.5, preferably from 4.5 to 6.0, and most preferably of 4.8 to 5.5. According to another embodiment the aqueous buffer solution has a concentration from 0.01 mol/kg to 2 mol/kg, preferably from 0.04 to 1.0 mol/kg, more preferably from 0.06 to 0.5 mol/kg, even more preferably from 0.07 to 0.4 mol/kg, and most preferably from 0.08 to 0.2 mol/kg.

It should be understood that for the purpose of the present invention, the following terms have the following meaning.

The term "abrasive material" in the meaning of the present invention refers to a particulate substance that is capable of polishing or cleaning surfaces by rubbing or exfoliating.

For the purpose of the present invention, an "acid" is defined as Brønsted-Lowry acid, that is to say, it is an H₃O⁺ ion provider. According to the present invention, the acid can also be an acid salt, generating H₃O⁺ ions under the preparation conditions. An "acid salt" is defined as an H₃O⁺ ion-provider that is partially neutralized by an electropositive element. A "salt" is defined as an electrically neutral ionic compound formed from anions and cations. A "partially crystalline salt" is defined as a salt that, on XRD analysis, presents an essentially discrete diffraction pattern.

In accordance with the present invention, pKₐ, is the symbol representing the acid dissociation constant associated with a given ionisable hydrogen in a given acid, and is indicative of the natural degree of dissociation of this hydrogen from this acid at equilibrium in water at a given temperature. Such pKₐ values may be found in reference textbooks such as Harris, D. C. "Quantitative Chemical Analysis: 3rd Edition", 1991, W.H. Freeman & Co. (USA), ISBN 0-7167-2170-8, or CRC Handbook of Chemistry and Physics, 1994-1995 75th edition, 8-43 to 8-55, CRC Press Inc., 1995.

The term "conjugate base" refers to a substance that is formed by removal of a proton from an acid. By gaining a proton the conjugate base is reformed into the acid.

"Natural ground calcium carbonate" (GCC) in the meaning of the present invention is a calcium carbonate obtained from natural sources, such as limestone, marble, or chalk, and processed through a wet and/or dry treatment such as grinding, screening and/or fractionation, for example, by a cyclone or classifier.

"Precipitated calcium carbonate" (PCC) in the meaning of the present invention is a synthesized material, generally obtained by precipitation following a reaction of carbon dioxide and calcium hydroxide (hydrated lime) in an aqueous environment or by precipitation of a calcium- and a carbonate source in water. Additionally, precipitated calcium carbonate can also be the product of introducing calcium and carbonate salts, calcium chloride and sodium carbonate for example, in an aqueous environment. PCC may have a vateritic, calcitic or aragonitic crystalline form. PCCs are described, for example, in EP 2 447 213 A1, EP 2 524 898 A1, EP 2 371 766 A1, or WO 2013/142473 A1.

For the purpose of the present invention, the terms "agglomerate" and "aggregate" shall have the definition as set out in ISO 14887:2000(E). There, an "agglomerate" is defined as an assemblage of particles which are loosely coherent, and an "aggregate" is defined as an assemblage of particles rigidly joined together.

Throughout the present document, the "particle size" of a filler material or other particulate material is described by its distribution of particle sizes. The value *dₓ* represents the diameter relative to which x % by volume of the particles have diameters less than *dₓ*. The *d*₅₀ value is thus the volume determined median particle size, i.e. 50 % of the total volume of all particles results from particles bigger and 50 % of the total volume of all particles results from particles smaller than this particle size. For the purpose of the present invention the particle size is specified as volume determined median particle size *d*₅₀ unless indicated otherwise. For determining the volume determined median particle size *d*₅₀ a Mastersizer 2000 or Mastersizer 3000 from the company Malvern Instruments Ltd., Great Britain, using the Fraunhofer light scattering model, may be used. The weight determined particle size distribution may correspond to the volume determined particle size if the density of all the particles is equal. The method and the instrument are known to the skilled person and are commonly used to determine grain size of fillers and pigments. The samples are dispersed using a high speed stirrer and supersonics.

For the purpose of the present invention, the "solids content" of a liquid composition is a measure of the amount of material remaining after all the solvent or water has been evaporated.

A "specific surface area (SSA)" of a calcium carbonate in the meaning of the present invention is defined as the surface area of the mineral pigment divided by the mass of the calcium carbonate. As used herein, the specific surface area is measured by nitrogen gas adsorption using the BET isotherm (ISO 9277:2010) and is specified in m²/g.

The term "surface-treated calcium carbonate" in the meaning of the present invention refers to a calcium carbonate which has been contacted with at least one acid and at least one conjugate base as defined in claim 1, such as to obtain a surface-treatment on at least a part of the surface of the calcium carbonate.

In the meaning of the present invention, the term "surfactant" refers to a compound that lowers the surface tension or interfacial tension between two liquids or between a liquid and a solid, and may act as detergent, wetting agent, emulsifier, foaming agent, or dispersant.

For the purpose of the present invention, the term "viscosity" or "Brookfield viscosity" refers to Brookfield viscosity. The Brookfield viscosity is for this purpose measured by a Brookfield DV-II+ Pro viscometer at 25°C ± 1°C at 100 rpm using an appropriate spindle of the Brookfield RV-spindle set and is specified in mPa·s. Based on his technical knowledge, the skilled person will select a spindle from the Brookfield RV-spindle set which is suitable for the viscosity range to be measured. For example, for a viscosity range between 200 and 800 mPa·s the spindle number 3 may be used, for a viscosity range between 400 and 1 600 mPa·s the spindle number 4 may be used, for a viscosity range between 800 and 3 200 mPa·s the spindle number 5 may be used, for a viscosity range between 1 000 and 2 000 000 mPa·s the spindle number 6 may be used, and for a viscosity range between 4 000 and 8 000 000 mPa·s the spindle number 7 may be used.

A "suspension" or "slurry" in the meaning of the present invention comprises insoluble solids and a solvent or liquid, preferably water, and optionally further additives, and usually contains large amounts of solids and, thus, is more viscous and can be of higher density than the liquid from which it is formed.

Where the term "comprising" is used in the present description and claims, it does not exclude other non-specified elements of major or minor functional importance. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Whenever the terms "including" or "having" are used, these terms are meant to be equivalent to "comprising" as defined above.

Where an indefinite or definite article is used when referring to a singular noun, e.g. "a", "an" or "the", this includes a plural of that noun unless something else is specifically stated.

Terms like "obtainable" or "definable" and "obtained" or "defined" are used interchangeably. This e.g. means that, unless the context clearly dictates otherwise, the term "obtained" does not mean to indicate that e.g. an embodiment must be obtained by e.g. the sequence of steps following the term "obtained" even though such a limited understanding is always included by the terms "obtained" or "defined" as a preferred embodiment.

The inventive process for producing a surface-treated calcium carbonate with improved stability in environments with a pH of 4.5 to 7 comprises the steps of: (i) providing calcium carbonate, (ii) providing at least one acid having a pKa value from 0 to 8, when measured at 20°C, (iii) providing at least one conjugate base, (iv) contacting the calcium carbonate of step (i), the at least one acid of step (ii), and the at least one conjugate base of step (iii) to form surface-treated calcium carbonate. The at least one acid of step (ii) and/or the at least one conjugate base of step (iii) are provided in form of an aqueous solution.

In the following details and preferred embodiments of the inventive process will be set out in more detail. It is to be understood that these technical details and embodiments also apply to the inventive surface-treated calcium carbonate obtained from said process, the inventive use of said inventive surface-treated calcium carbonate and compositions comprising said inventive compacted material.

### The calcium carbonate

In step i) of the process of the present invention, calcium carbonate is provided. According to one embodiment, the calcium carbonate of step i) is natural ground calcium carbonate, precipitated calcium carbonate, dolomite, agglomerates or aggregates thereof, or a mixture of the aforementioned materials. Preferably, the calcium carbonate of step i) is natural ground calcium carbonate.

Natural ground calcium carbonate (GCC) is understood to be manufactured from a naturally occurring form of calcium carbonate, mined from sedimentary rocks such as limestone or chalk, or from metamorphic marble rocks, eggshells or seashells. Calcium carbonate is known to exist as three types of crystal polymorphs: calcite, aragonite and vaterite. Calcite, the most common crystal polymorph, is considered to be the most stable crystal form of calcium carbonate. Less common is aragonite, which has a discrete or clustered needle orthorhombic crystal structure. Vaterite is the rarest calcium carbonate polymorph and is generally unstable. Ground calcium carbonate is almost exclusively of the calcitic polymorph, which is said to be trigonal-rhombohedral and represents the most stable of the calcium carbonate polymorphs. The term "source" of the calcium carbonate in the meaning of the present application refers to the naturally occurring mineral material from which the calcium carbonate is obtained. The source of the calcium carbonate may comprise further naturally occurring components such as magnesium carbonate, alumino silicate etc.

According to one embodiment of the present invention the source of natural ground calcium carbonate (GCC) is selected from marble, chalk, limestone, or mixtures thereof. Preferably, the source of natural ground calcium carbonate is marble. According to one embodiment of the present invention the GCC is obtained by dry grinding. According to another embodiment of the present invention the GCC is obtained by wet grinding and subsequent drying.

"Dolomite" in the meaning of the present invention is a calcium carbonate containing mineral, namely a carbonic calcium-magnesium-mineral, having the chemical composition of CaMg(CO₃)₂ ("CaCO₃ · MgCO₃"). A dolomite mineral may contain at least 30.0 wt.-% MgCO₃ based on the total weight of dolomite, preferably more than 35.0 wt.-%, and more preferably more than 40.0 wt.-% MgCO₃.

According to one embodiment of the present invention, the calcium carbonate comprises one type of natural ground calcium carbonate. According to another embodiment of the present invention, the calcium carbonate comprises a mixture of two or more types of natural ground calcium carbonates selected from different sources.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source in water or by precipitation by combining calcium and carbonate ions, for example CaCl₂ and Na₂CO₃, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the calcium carbonate comprises one type of precipitated calcium carbonate. According to another embodiment of the present invention, the calcium carbonate comprises a mixture of two or more precipitated calcium carbonates selected from different crystalline forms and different polymorphs of precipitated calcium carbonate. For example, the at least one precipitated calcium carbonate may comprise one PCC selected from S-PCC and one PCC selected from R-PCC. According to still another embodiment of the present invention, the calcium carbonate comprises agglomerates or aggregates of precipitated calcium carbonate having a volume determined median particle size *d*₅₀ from 25 to 1 500 µm.

According to one embodiment of the present invention, the calcium carbonate is a natural ground calcium carbonate. According to another embodiment of the present invention, the calcium carbonate is a precipitated calcium carbonate. According to still another embodiment of the present invention, the calcium carbonate is a mixture of natural ground calcium carbonate and precipitated calcium carbonate. According to still another embodiment of the present invention, the calcium carbonate is dolomite, optionally in mixture with natural ground calcium carbonate and/or precipitated calcium carbonate.

According to one embodiment, the calcium carbonate of step i) is in form of particles having a volume determined median particle size *d*₅₀ from 1 to 1 500 µm, preferably from 25 to 1 400 µm, more preferably from 100 from 1 200 µm, even more preferably from 200 to 1 000 µm, and most preferably from 300 to 800 µm.

According to one embodiment the calcium carbonate of step i) is selected from the group consisting of natural ground calcium carbonate, dolomite, aggregates or agglomerates of PCC, or mixtures of the aforementioned, and is in form of particles having a volume determined median particle size *d*₅₀ from 25 to 1 500 µm, preferably from 25 to 1 400 µm, more preferably from 100 to 1 200 µm, even more preferably from 150 from 1 100 µm, still more preferably from 200 to 1 000 µm, and most preferably from 300 to 800 µm.

According to one embodiment, the calcium carbonate of step i) is in form of particles having a volume determined top cut particle size (*d*₉₈) from 40 to 2 000 µm, preferably from 200 to 1 800 µm, more preferably from 300 to 1 500 µm, even more preferably from 400 to 1 200 µm, and most preferably from 500 to 1 000 µm.

According to another embodiment, the calcium carbonate of step i) is in form of particles having a specific BET surface area in the range of > 0 to 5 m²/g, preferably in the range of > 0 to 2 m²/g, and more preferably in the range of > 0 to 1.6 m²/g.

The calcium carbonate may be used in any suitable liquid or dry form. For example, the calcium carbonate may be in form of a powder and/or a suspension. The suspension can be obtained by mixing particles of calcium carbonate with a solvent, preferably water. The calcium carbonate to be mixed with a solvent, and preferably water, may be provided in any form, for example, as suspension, slurry, dispersion, paste, powder, a moist filter cake or in pressed or granulated form.

The suspension can be undispersed or dispersed, i.e. the suspension includes a dispersant, and thus, forms an aqueous dispersion.

According to one embodiment of the present invention, the calcium carbonate is used in form of an aqueous suspension, which does not contain a dispersant. According to another embodiment of the present invention, calcium carbonate is used in form of an aqueous suspension, which contains a dispersant. A suitable dispersant may be selected from polyphosphates, and is in particular a tripolyphosphate. Another suitable dispersant may be selected from the group comprising homopolymers or copolymers of polycarboxylic acid salts based on, for example, acrylic acid, methacrylic acid, maleic acid, fumaric acid or itaconic acid and acrylamide or mixtures thereof. Homopolymers or copolymers of acrylic acid are especially preferred. The weight average molecular weight M_{w} of such products is preferably in the range from 2 000 to 15 000 g/mol, with a weight average molecular weight M_{w} from 3 000 to 7 000 g/mol or 3 500 to 6 000 g/mol being especially preferred. According to an exemplary embodiment, the dispersant is sodium polyacrylate having a weight average molecular weight M_{w} from 2 000 to 15 000 g/mol, preferably from 3 000 to 7 000 g/mol, and most preferably from 3 500 to 6 000 g/mol.

The solids content of the suspension of calcium carbonate can be adjusted by the methods known to the skilled person. To adjust the solids content of an aqueous suspension, for example, the aqueous suspension may be partially dewatered by a settling, filtration, centrifugation or thermal separation process. According to one embodiment of the present invention, the solids content of the aqueous suspension of calcium carbonate is from 1 to 85 wt.-%, more preferably from 5 to 75 wt.-%, and most preferably from 10 to 65 wt.-%, based on the total weight of the aqueous suspension.

### The at least one acid and the at least one conjugate base

In process step ii) at least one acid is provided having a pKₐ value from 0 to 8, when measured at 20°C.

The at least one acid of step ii) can be any medium-strong acid, or weak acid, or mixtures thereof, generating H₃O⁺ ions under the preparation conditions. According to the present invention, the at least one acid can also be an acidic salt, generating H₃O⁺ ions under the preparation conditions.

The at least one acid of step ii) and the at least one conjugate base of step iii), when available in dry form, may include crystalline water.

According to one embodiment, the at least one acid of step ii) is a medium-strong acid having a pKₐ value from 0 to 2.5, when measured at 20°C. If the pKₐ at 20°C is from 0 to 2.5, the acid is preferably selected from sulphurous acid, phosphoric acid, oxalic acid, or mixtures thereof, more preferably from phosphoric acid, oxalic acid, or mixtures thereof, and most preferably from phosphoric acid. The at least one acid can also be an acidic salt, for example, H₂PO₄⁻, being at least partially neutralised by a corresponding cation such as Li⁺, Na⁺ or K⁺, or HPO₄²⁻, being at least partially neutralised by a corresponding cation such as Li⁺, Na⁺, K⁺, Mg²⁺ or Ca²⁺. The at least one acid can also be a mixture of one or more acids and one or more acidic salts.

According to still another embodiment, the at least one acid is a weak acid having a pKₐ value of greater than 2.5 and less than or equal to 7, when measured at 20°C. According to the preferred embodiment, the weak acid has a pKₐ value from 2.6 to 5 at 20°C, and more preferably the weak acid is selected from the group consisting of acetic acid, formic acid, propanoic acid, citric acid, boric acid, tartaric acid, phosphonic acid, and mixtures thereof, preferably from the group consisting of citric acid, tartaric acid, and mixtures thereof, and is most preferably citric acid.

According to one embodiment of the present invention, the at least one acid of step ii) is selected from the group consisting of phosphoric acid, citric acid, sulphurous acid, boric acid, acetic acid, tartaric acid, formic acid, propanoic acid, oxalic acid, phosphonic acid, acidic salt, and mixtures thereof, preferably selected from the group consisting of phosphoric acid, citric acid, tartaric acid, oxalic acid, their acidic salts, and mixtures thereof, and most preferably selected from the group consisting of phosphoric acid, citric acid, their acidic salts, and mixtures thereof.

Preferably the at least one acid of step ii) may be a phosphoric acid or acidic salt thereof, a citric acid or acidic salt thereof, or a mixture thereof. According to a preferred embodiment, the at least one acid is selected from the group consisting of phosphoric acid, citric acid, sodium dihydrogen phosphate, potassium dihydrogen phosphate, potassium dihydrogen citrate, sodium dihydrogen citrate, disodium hydrogen citrate, dipotassium hydrogen citrate and mixtures thereof.

It is preferred that when the used acid(s) is an organic acid, then it is preferably not an aliphatic monocarboxylic acid derived from an animal or vegetable fat, oil or wax, and having a chain of 4 to 28 carbons (usually branched and even-numbered), which may be saturated or unsaturated. It has to be noted that this is the IUPAC definition of fatty acids. In other words, if an organic acid is used in the process of the present invention, then it is preferably not a fatty acid. The aforementioned also applies to the conjugate base.

The at least one acid of step ii) can be provided in liquid or dry form. For example, the at least one acid may be in form of a solid such as granules or a powder and/or a solution. According to one embodiment, the at least one acid of step ii) is provided in form of an aqueous solution. When the at least one acid is in form of a solid such as granules or a powder, the acid may include crystalline water.

The at least one acid of step ii) can be provided as a concentrated solution or a more diluted solution. Preferably, the molar ratio of the at least one acid to the calcium carbonate is from 1:10 to 1:100, preferably from 1:20 to 1:80, and more preferably from 1:50 to 1:60.

According to one embodiment of the present invention, the aqueous solution of the at least one acid of step ii) has a concentration from 0.01 mol/kg to 2 mol/kg, preferably from 0.04 to 1.0 mol/kg, more preferably from 0.06 to 0.5 mol/kg, even more preferably from 0.07 to 0.4 mol/kg, and most preferably from 0.08 to 0.2 mol/kg.

In process step iii) at least one conjugate base is provided. A conjugate base according to the present invention is a substance that is formed by removal of a proton from an acid. By gaining a proton the conjugate base is reformed into the corresponding acid.

The at least one conjugate base of step iii) can be an alkali metal salt and/or alkaline earth metal salt of an acid. According to one embodiment of the present invention, the at least one conjugate base is an alkali metal salt and/or alkaline earth metal salt of an acid being selected from the group consisting of phosphoric acid, citric acid, sulphurous acid, boric acid, acetic acid, tartaric acid, formic acid, propanoic acid, oxalic acid, phosphonic acid, and mixtures thereof. The alkali metal may be selected from lithium, sodium, or potassium. The alkaline earth metal may be magnesium.

Preferably the at least one conjugate base of step iii) may be an alkali metal salt and/or alkaline earth metal salt of an acid being selected from a phosphoric acid or acidic salt thereof, a citric acid or acidic salt thereof, or a mixture thereof. According to a preferred embodiment, the at least one conjugate base of step iii) is disodium hydrogen phosphate, dipotassium hydrogen phosphate, tripotassium citrate and/or trisodium citrate.

According to one embodiment, the at least one conjugate base of step iii) is a conjugate base of the at least one acid of step ii). For example, if the at least one acid of step ii) is phosphoric acid, the at least one conjugate base of step iii) is an alkali metal phosphate and/or alkaline earth metal phosphate. According to another exemplary embodiment, if the at least one acid of step ii) is citric acid, the at least one conjugate base of step iii) is an alkali metal citrate and/or alkaline earth metal citrate. However, the at least one conjugate base of step iii) can also be a conjugate base of an acid different from the at least one acid of step ii). For example, the at least one acid of step ii) can be citric acid and the at least one conjugate base of step iii) can be an alkali metal phosphate and/or alkaline earth metal phosphate.

The at least one conjugate base of step iii) can be provided in liquid or dry form. For example, the at least one conjugate base may be in form of a solid such as granules or a powder and/or a solution. According to one embodiment, the at least one conjugate base of step iii) is provided in form of an aqueous solution.

The at least one conjugate base of step iii) can be provided as a concentrated solution or a more diluted solution. Preferably, the molar ratio of the at least one conjugate base to the calcium carbonate is from 1:10 to 1:100, preferably from 1:20 to 1:80, and more preferably from 1:50 to 1:60.

According to one embodiment of the present invention, the aqueous solution of the at least one conjugate base of step iii) has a concentration from 0.01 mol/kg to 2 mol/kg, preferably from 0.04 to 1.0 mol/kg, more preferably from 0.06 to 0.5 mol/kg, even more preferably from 0.07 to 0.4 mol/kg, and most preferably from 0.08 to 0.2 mol/kg.

The at least one acid of process step ii) and the at least one conjugate base of process step iii) can be provided separately or in combination.

According to the present invention, the at least one acid of step ii) and/or the at least one conjugate base of step iii) are provided in form of an aqueous solution. According to one embodiment, the at least one acid of step ii) as well as the at least one conjugate base of step iii) are provided as separate aqueous solutions.

According to one embodiment, the at least one acid of step ii) and the at least one conjugate base of step iii) are provided together in form of an aqueous buffer solution. According to one embodiment of the present invention the aqueous buffer solution is selected from the group consisting of phosphate buffer, phosphate-citrate buffer, citrate buffer, tartrate buffer, borate buffer, preferably the aqueous buffer solution is a phosphate buffer, a phosphate-citrate buffer, or a citrate buffer, and more preferably a phosphate-citrate buffer, or a citrate buffer.

According to a preferred embodiment, the at least one acid of step ii) and the at least one conjugate base of step iii) are provided together in form of an aqueous buffer solution, wherein the at least one acid of step ii) is selected from sodium dihydrogen phosphate, potassium dihydrogen phosphate, citric acid, sodium dihydrogen citrate, potassium dihydrogen citrate, disodium hydrogen citrate, dipotassium hydrogen citrate, or mixtures thereof, and the at least one conjugate base of step iii) is disodium hydrogen phosphate, dipotassium hydrogen phosphate, tripotassium citrate and/or trisodium citrate. More preferably, the at least one acid of step ii) may be selected from sodium dihydrogen phosphate, sodium dihydrogen citrate, or mixtures thereof, and the at least one conjugate base of step iii) may be disodium hydrogen phosphate, dipotassium hydrogen phosphate, tripotassium citrate and/or trisodium citrate.

According to one embodiment the aqueous buffer solution has a pH value from 4.0 to 7.5, preferably from 4.5 to 6.0, and most preferably from 4.8 to 5.5. According to another embodiment the aqueous buffer solution of step has a concentration from 0.01 mol/kg to 2 mol/kg, preferably from 0.04 to 1.0 mol/kg, more preferably from 0.06 to 0.5 mol/kg, even more preferably from 0.07 to 0.4 mol/kg, and most preferably from 0.08 to 0.2 mol/kg.

According to one embodiment the aqueous buffer solution is provided in an amount from 0.1 to 99 wt.-%, based on the total amount of the aqueous suspension of calcium carbonate, preferably in an amount from 1 to 90 wt.-%, more preferably in an amount from 10 to 80 wt.-%, and most preferably from 60 to 70 wt.-%.

### The process for producing a surface-treated calcium carbonate

According to one aspect of the present invention, a process for producing a surface-treated calcium carbonate with improved stability in environments with a pH of 4.5 to 7 is provided, wherein the process comprises the steps of:
i) providing calcium carbonate,
ii) providing at least one acid having a pKₐ value from 0 to 8, when measured at 20°C
iii) providing at least one conjugate base,
iv) contacting the calcium carbonate of step i), the at least one acid of step ii), and the at least one conjugate base of step iii) to form surface-treated calcium carbonate,
wherein the at least one acid of step ii) and/or the at least one conjugate base of step iii) are provided in form of an aqueous solution.

According to step iv) of the inventive process, the calcium carbonate of step i), the at least one acid of step ii), and the at least one conjugate base of step iii) are contacted.

The calcium carbonate of step i) may be contacted with the at least one acid of step ii) and the at least one conjugate base of step iii) in one step or in several steps.

According to one embodiment of the present invention, step iv) comprises the steps of:
a1) contacting the calcium carbonate of step i) and the at least one acid of step ii) to form a pre-treated calcium carbonate, and
a2) contacting the pre-treated calcium carbonate of step a1) with the at least one conjugate base of step iii) to form surface-treated calcium carbonate,
wherein at least the at least one acid of step ii) is provided in form of an aqueous solution.

According to another embodiment of the present invention, step iv) comprises the steps of:
b1) contacting the calcium carbonate of step i) and the at least one conjugate base of step iii) to form a pre-treated calcium carbonate, and
b2) contacting the pre-treated calcium carbonate of step b1) with the at least one acid of step ii) to form surface-treated calcium carbonate,
wherein at least the at least one conjugate base of step iii) is provided in form of an aqueous solution.

Alternatively, the calcium carbonate of step i), the at least one acid of step ii) and the at least one conjugate base of step iii) are contacted simultaneously. In this case, the at least one acid of step ii) and the at least one conjugate base of step iii) may be provided separately or as a mixture. Preferably, the at least one acid of step ii) and the at least one conjugate base of step iii) may be provided as a mixture, for example, in form of an aqueous buffer solution comprising the at least acid of step ii) and the at least one conjugate base of step iii).

According to one embodiment of the present invention, the at least one acid of step ii) and the at least one conjugate base of step iii) are provided together in form of an aqueous buffer solution, and in step iv) the calcium carbonate of step i) is contacted with the aqueous buffer solution to form surface-treated calcium carbonate.

The contacting step iv) can be carried out by any means known in the art. For example, the calcium carbonate of step i), the at least one acid of step ii), and the at least one conjugate base of step iii) can be brought into contact by spraying and/or mixing. Suitable process equipment for spraying or mixing is known to the skilled person.

According to one embodiment of the present invention, process step iv) is carried out by spraying. According to another embodiment of the present invention, process step iv) is carried out by mixing.The skilled person will adapt the mixing conditions such as the mixing speed and temperature according to his process equipment. For example, the mixing may take place by means of a ploughshare mixer. Ploughshare mixers function by the principle of a fluidized bed produced mechanically. Ploughshare blades rotate close to the inside wall of a horizontal cylindrical drum and convey the components of the mixture out of the product bed and into the open mixing space. The fluidized bed produced mechanically ensures intense mixing of even large batches in a very short time. Choppers and/or dispersers are used to disperse lumps in a dry operation. Equipment that may be used in the inventive process is available, for example, from Gebrüder Lödige Maschinenbau GmbH, Germany. Furthermore, a tubular mixing apparatus, for example, from Ystral GmbH, Ballrechten-Dottingen, Germany may be used.

Process step iv) may be carried out at room temperature, i.e. at a temperature of 20°C ± 2°C, or at other temperatures. According to one embodiment of the present invention, process step iv) is carried out for at least 1 s, preferably for at least one min, e.g. for at least 2 min. According to one embodiment, process step iv) is carried out by mixing for at least 1 min, preferably at least 5 min, more preferably at least 10 min, and most preferably at least 20 min.

According to the present invention the at least one acid of step ii) and/or the at least one conjugate base of step iii) are provided in form of an aqueous solution. Depending on the amount of water that is introduced during step iv) by contacting the aforementioned compounds, the surface-treated calcium carbonate is obtained in form of a wet or moist surface-treated calcium carbonate or in form of an aqueous suspension of surface-treated calcium carbonate. It is also within the confines of the present invention that additional water may be introduced during process step iv), for example, in order to control and/or maintain and/or achieve the desired solids content or Brookfield viscosity of the obtained wet or moist surface-treated calcium carbonate or aqueous suspension of surface-treated calcium carbonate.

By means of step iv), a suspension of surface-treated calcium carbonate is obtained, the suspension having a solid content in the range from 1 to 80 wt.-%, more preferably in the range from 10 to 60 wt.-%, and most preferably in the range from 20 to 50 wt.-%, based on the weight of the surface-treated calcium carbonate in the suspension.

According to the present invention, the process can further comprise a step v) of drying the surface-treated calcium carbonate.

In general, the drying step v) may take place using any suitable drying equipment and can, for example, include thermal drying and/or drying at reduced pressure using equipment such as an evaporator, a flash drier, an oven, a spray drier and/or drying in a vacuum chamber.

According to one embodiment, drying step v) is a spray drying step, preferably said spray drying step is carried out with an inlet temperature of 100°C - 1000°C and preferably from 200°C to 600°C. Spray drying may be preferred in case of small particle sizes, e.g., at a volume determined median particle size *d*₅₀ from 1 to 20 µm In case of a volume determined median particle size *d*₅₀ of more than 20 µm other drying methods, e.g., drying in an oven, fluid bed dryer or flash drying may be preferred.

By means of drying step v), a dried surface-treated calcium carbonate is obtained, preferably having a low total moisture content. According to one embodiment, the surface-treated calcium carbonate obtained by step v) has a total moisture content which is less than or equal to 1.0 wt.-%, based on the total weight of the dried surface-treated calcium carbonate.

According to another embodiment, the dried surface-treated calcium carbonate obtained by step v) has a total moisture content of less than or equal to 0.5 wt.-%, and preferably less than or equal to 0.2 wt.-%, based on the total weight of the dried surface-treated calcium carbonate. According to still another embodiment, the dried surface-treated calcium carbonate obtained by step v) has a total moisture content of between 0.01 and 0.15 wt.-%, preferably between 0.02 and 0.10 wt.-%, and more preferably between 0.03 and 0.07 wt.-%, based on the total weight of the dried surface-treated calcium carbonate.

According to one optional embodiment of the present invention, after step iv) and before step v), the surface-treated calcium carbonate is left to stand for at least 6 h, preferably at least 12 h, more preferably at least 24 h, and most preferably at least 48 h. This prolonged contacting time may improve the efficiency of the surface treatment and may lead to the formation of a more homogeneous surface-treatment layer.

In case an aqueous suspension of surface-treated calcium carbonate is obtained after step iv), it may be necessary to separate the surface-treated calcium carbonate from the aqueous suspension. According to an optional embodiment, in process step iv) the calcium carbonate of step i), the at least one acid of step ii), the at least one conjugate base of step iii), and optionally water, are contacted to form an aqueous suspension of surface-treated calcium carbonate, and after step iv) and before step v) the surface-treated calcium carbonate is separated from the aqueous suspension obtained from step iv).

According to one embodiment, the calcium carbonate of step i) is provided in an amount from 10 to 99 wt.-%, based on the total amount of the aqueous suspension of surface-treated calcium carbonate, preferably in an amount from 10 to 95 wt.-%, more preferably in an amount from 20 to 90 wt.-%, and most preferably in an amount from 30 to 40 wt.-%.

According to one embodiment of the present invention, the at least one acid of step ii) and the at least one conjugate base of step iii) are provided together in form of an aqueous buffer solution, and step iv) comprises the steps of:
c1) mixing the calcium carbonate of step i), the aqueous buffer solution, and optionally water, to form an aqueous suspension of surface-treated calcium carbonate, and
c2) separating the surface-treated calcium carbonate from the aqueous suspension obtained from step c1).

For the purpose of the present invention, the expression "separating" means that the surface-treated calcium carbonate is removed or isolated from the aqueous suspension obtained from step c1) of the inventive process. The surface-treated calcium carbonate obtained from step c1) may be separated from the aqueous suspension by any conventional means of separation known to the skilled person. According to one embodiment of the present invention, in process step c2) the surface-treated calcium carbonate is separated mechanically and/or thermally. Examples for mechanical separation processes are filtration, e.g. by means of a drum filter or filter press, nanofiltration, or centrifugation. An example for a thermal separation process is a concentrating process by the application of heat, for example, in an evaporator. According to a preferred embodiment, in process step c2) the surface-treated calcium carbonate is separated mechanically, preferably by filtration and/or centrifugation.

According to one embodiment, after step iv) and before step v), the surface-treated calcium carbonate is washed with a washing solution, preferably water. For example, the surface-treated calcium carbonate can be washed one time, two times or three times with a washing solution, preferably water. Suitable washing solutions may be selected from water, ethanol, or mixtures thereof.

According to a preferred embodiment of the present invention, a process for producing a surface-treated calcium carbonate with improved stability in environments with a pH of 4.5 to 7 is provided, wherein the process comprises the steps of:
I) providing calcium carbonate,
II) providing an aqueous buffer solution comprising at least one acid having a pKₐ value from 0 to 8, when measured at 20°C, and at least one conjugate base,
III) contacting the calcium carbonate of step I), the buffer solution of step II), and optionally water, to form surface-treated calcium carbonate.

According to one embodiment the aqueous buffer solution is selected from the group consisting of phosphate buffer, phosphate-citrate buffer, citrate buffer, tartrate buffer, borate buffer, preferably the aqueous buffer solution is a phosphate buffer, a phosphate-citrate buffer, or a citrate buffer, and more preferably a phosphate-citrate buffer, or a citrate buffer. According to a preferred embodiment, the at least one acid is selected from sodium dihydrogen phosphate, potassium dihydrogen phosphate, citric acid, sodium dihydrogen citrate, potassium dihydrogen citrate, disodium hydrogen citrate, dipotassium hydrogen citrate, or mixtures thereof, and the at least one conjugate base is disodium hydrogen phosphate, dipotassium hydrogen phosphate, tripotassium citrate and/or trisodium citrate. More preferably, the at least one acid may be selected from sodium dihydrogen phosphate, potassium dihydrogen phosphate, sodium dihydrogen citrate, potassium dihydrogen citrate, or mixtures thereof, and the at least one conjugate base may be disodium hydrogen phosphate, dipotassium hydrogen phosphate, tripotassium citrate and/or trisodium citrate.

According to the present invention, this process can further comprise a step IV) of drying the surface-treated calcium carbonate as described above with respect to drying step v).

### The surface-treated calcium carbonate

According to a further aspect of the present invention, a suspension of a surface-treated calcium carbonate with improved stability in environments with a pH of 4.5 to 7 is provided, which is obtainable by a process comprising the steps of:
i) providing calcium carbonate,
ii) providing at least one acid having a pKₐ value from 0 to 8, when measured at 20°C,
iii) providing at least one conjugate base,
iv) contacting the calcium carbonate of step i), the at least one acid of step ii), and the at least one conjugate base of step iii) to form surface-treated calcium carbonate,
wherein the at least one acid of step ii) and/or the at least one conjugate base of step iii) are provided in form of an aqueous solution.

According to a further aspect of the present invention, a dried surface-treated calcium carbonate with improved stability in environments with a pH of 4.5 to 7 is provided, which is obtainable by a process comprising the steps of:
i) providing calcium carbonate,
ii) providing at least one acid having a pKₐ value from 0 to 8, when measured at 20°C,
iii) providing at least one conjugate base,
iv) contacting the calcium carbonate of step i), the at least one acid of step ii), and the at least one conjugate base of step iii) to form surface-treated calcium carbonate, and
v) drying the surface-treated calcium carbonate,
wherein the at least one acid of step ii) and/or the at least one conjugate base of step iii) are provided in form of an aqueous solution.

The inventors have surprisingly found that by implementing the process according to the present invention, a surface-treated calcium carbonate can be prepared that shows a similar BET surface area like the calcium carbonate that has been used for its preparation. In other words, the inventors have surprisingly found that the treatment of the present invention does not lead to or only leads to a minor increase of the BET surface of the treated product.

The following paragraphs are intended to refer to both the suspension of a surface-treated calcium carbonate with improved stability in environments with a pH of 4.5 to 7 and the dried surface-treated calcium carbonate with improved stability in environments with a pH of 4.5 to 7, unless otherwise stated.

According to one embodiment the surface-treated calcium carbonate is in form of particles having a volume determined median particle size *d*₅₀ from 1 to 1 500 µm, preferably from 25 to 1 400 µm, more preferably from 100 to 1 200 µm, even more preferably from 200 to 1 000 µm, and most preferably from 300 to 800 µm. Additionally or alternatively, the surface-treated calcium carbonate may be in form of particles having a volume determined top cut particle size *d*₉₈ from 40 to 2 000 µm, preferably from 200 to 1 800 µm, more preferably from 300 to 1 500 µm, even more preferably from 400 to 1 200 µm, and most preferably from 500 to 1 000 µm.

According to one embodiment the surface-treated calcium carbonate has a reduced acid consumption per hour at pH 5 and pH 6 when treated with acetic acid, the reduction being at least 25 %, more preferably at least 50 %, and most preferably at least 70 %.

According to another embodiment the surface-treated calcium carbonate is in form of particles having a specific BET surface area in the range of > 0 to 5 m²/g, preferably in the range of > 0 to 2 m²/g, and more preferably in the range of > 0 to 1.6 m²/g.

The surface-treated calcium carbonate obtainable by the process of the present invention may be used for various applications. According to one aspect of the present invention, the surface-treated calcium carbonate is used as abrasive material. The inventors of the present invention found that the surface-treated calcium carbonate obtained by the process of the present invention may provide a very gentle abrasiveness, which makes it suitable for delicate surfaces and especially cosmetic applications. According to a preferred embodiment, the dried surface-treated calcium carbonate obtainable by the process of the present invention is used as abrasive material for cosmetic application and/or cleaning application, more preferably as abrasive material for cosmetic application, and most preferably as abrasive material for topical skin application. According to another preferred embodiment, the suspension of the surface-treated calcium carbonate obtainable by the process of the present invention is used as abrasive material for cleaning application.

The inventors of the present invention surprisingly found that the surface-treated calcium carbonate obtainable by the inventive process exhibits an improved stability at environments with a pH of 4.5 to 7. Therefore, the surface-treated calcium carbonate is especially suitable for applications in such environments. However, the inventive surface-treated calcium carbonate, in suspension or in dry from, can also be used in applications requiring pH values of > 7, for example pH values in the range from > 7 to 10 or 11. Furthermore, it was found that the surface-treated calcium carbonate obtained by the inventive process can replace conventional abrasive materials such as microbeads in abrasive cleaning compositions without affecting the sensory properties of the composition. Moreover, the dried surface-treated calcium carbonate is a non-toxic material and biologically degradable, which makes it, for example, most suitable for cosmetic applications and eco-friendly household cleaners.

### The abrasive cleaning composition

According to a further aspect of the present invention, an abrasive cleaning composition comprising a surface-treated calcium carbonate obtainable by a process according to the present invention is provided.

According to one embodiment, the abrasive cleaning composition comprises at least 1 wt.-%, based on the total weight of the composition, of the surface-treated calcium carbonate as an abrasive material. For example, the abrasive cleaning composition can comprise at least 2 wt.-%, at least 5 wt.-%, or at least 8 wt.-%, based on the total weight of the composition, of the surface-treated calcium carbonate as an abrasive material. According to one embodiment of the present invention, the composition comprises from 10 to 80 wt.-%, preferably from 15 to 70 wt.-%, more preferably from 17 to 60 wt.-%, even more preferably from 19 to 50 wt.-%, and most preferably about 20 wt.-% of the surface-treated calcium carbonate, based on the total weight of the composition.

The surface-treated calcium carbonate can consist of only one type of surface-treated calcium carbonate or can be a mixture of two or more types of surface-treated calcium carbonate. The abrasive cleaning composition of the present invention may contain the surface-treated calcium carbonate as the only abrasive material. Alternatively, the abrasive cleaning composition of the present invention may contain the surface-reacted calcium carbonate in combination with at least one additional abrasive material. The further abrasive material can be made from plastic, hard waxes, inorganic and organic abrasives, or natural materials.

According to one embodiment, the abrasive cleaning composition further comprises at least one additional abrasive material, preferably selected from the group consisting of silica, precipitated silica, alumina, aluminosilicate, metaphosphate, tricalcium phosphate, calcium pyrophosphate, natural ground calcium carbonate, precipitated calcium carbonate, sodium bicarbonate, bentonite, kaolin, aluminium hydroxide, calcium hydrogen phosphate, hydroxylapatite, apricot kernels, plum kernels, salt, sugar, polyethylene beads, luffa, rice, bamboo, microcrystalline cellulose, basalt, waxes, coffee, polylactic acid, cocoa exfoliator and mixtures thereof.

According to one embodiment, the additional abrasive material can have the same volume determined median particle size *d*₅₀ as the surface-treated calcium carbonate, namely a *d*₅₀ from 1 to 1 500 µm, preferably from 25 to 1 400 µm, more preferably from 100 from 1 200 µm, even more preferably from 200 to 1 000 µm, and most preferably from 300 to 800 µm. This abrasive material is suitable for topical applications.

Alternatively, the additional abrasive material has volume determined median particle size *d*₅₀ from 0.1 to 100 µm, preferably from 0.5 to 50 µm, more preferably from 1 to 20 µm, and most preferably from 2 to 10 µm. Such an abrasive material is suitable for oral applications.

The at least one additional abrasive material can be present in the abrasive cleaning composition in an amount from 1 to 80 wt.-%, based on the total weight of the composition, preferably from 5 to 70 wt.-%, more preferably from 10 to 60 wt.-%, and most preferably from 20 to 50 wt.-%.

The abrasive cleaning composition of the present invention may be in form of a solid or in form of a liquid. According to one embodiment, the abrasive cleaning composition is in form of a solid, preferably a powder, a granulate, a tablet, a stick, a chunk, a block, a sponge or a brick. According to another embodiment, the abrasive cleaning composition is in form of a liquid, preferably a solution, a suspension, a paste, an emulsion, a cream, an oil, or a gel.

According to one embodiment of the present invention, the abrasive cleaning composition is a liquid, aqueous composition. According to one embodiment the aqueous composition comprises from 1 to 90 wt.-% water, based on the total weight of the composition, preferably from 5 to 80 wt.-%, more preferably from 10 to 70, and most preferably from 20 to 60 wt.-%.

According to another embodiment of the present invention, the abrasive cleaning composition is a liquid, non-aqueous composition. According to one embodiment the aqueous composition comprises from 1 to 90 wt.-% of a solvent, based on the total weight of the composition, preferably from 5 to 80 wt.-%, more preferably from 10 to 70, and most preferably from 20 to 60 wt.-%. Suitable solvents are known to the skilled person and are, for example, aliphatic alcohols, ethers and diethers having from 4 to 14 carbon atoms, glycols, alkoxylated glycols, glycol ethers, alkoxylated aromatic alcohols, aromatic alcohols, terpenes, natural oils, or mixtures thereof.

According to one embodiment the abrasive cleaning composition has a pH from 5.0 to 7.0, measured at 20°C, preferably from 5.5 to 6.5, and most preferably of about 6. According to an alternative embodiment, the abrasive cleaning composition has a pH above 5, measured at 20°C. According to another alternative embodiment, the composition has a pH above 7.0, measured at 20°C. For example, the abrasive cleaning composition may have a pH from 7.5 to 11 or from 7.5 to 10. Means for adjusting the pH are known to the skilled person.

In case the abrasive cleaning composition is in liquid form, it may be a thickened composition having a Brookfield viscosity from 4 000 to 50 000 mPa·s at 20°C.

The abrasive cleaning composition may further comprise a surfactant. According to one embodiment of the present invention, the abrasive cleaning composition comprises from 0.1 to 30 wt.-% of a surfactant, preferably from 0.5 to 25 wt.-%, and most preferably from 2 to 20 wt.-%, based on the total amount of the composition.

Suitable surfactants are known to the skilled person and may be selected from non-ionic, anionic, zwitterionic, amphoteric, cationic surfactants, and mixtures thereof.

Examples of suitable nonionic surfactants are compounds produced by the condensation of simple alkylene oxides, which are hydrophilic in nature, with an aliphatic or alkyl-aromatic hydrophobic compound having a reactive hydrogen atom. Suitable anionic surfactants are, for example, water-soluble salts of organic sulphuric acid mono-esters and sulphonic acids which have in the molecular structure a branched or straight chain alkyl group containing from 6 to 22 carbon atoms in the alkyl part. Examples of suitable zwitterionic surfactants are of aliphatic quaternary ammonium, sulphonium and phosphonium compounds having an aliphatic group of from 8 to 18 carbon atoms and an aliphatic group substituted by an anionic water-solubilising group, for instance betaine and betaine derivatives such as alkyl betaine, in particular C₁₂-C₁₆ alkyl betaine, 3-(N,N-dimethyl-N- hexadecylammonium)-propane-1-sulphonate betaine, 3-(dodecylmethyl-sulphonium)- propane-1-sulphonate betaine, 3-(cetylmethyl-phosphonium)-propane-1-sulphonate betaine and N,N-dimethyl-N-dodecyl-glycine. Suitable amphoteric surfactants are, for example, derivatives of aliphatic secondary and tertiary amines containing an alkyl group of 8 to 20 carbon atoms and an aliphatic group substituted by an anionic water-solubilising group, for instance sodium 3-dodecylamino-propionate, sodium 3-dodecylaminopropane-sulphonate and sodium N-2-hydroxy-dodecyl-N-methyltaurate. Examples of suitable cationic surfactants are quaternary ammonium salts having one or two alkyl or aralkyl groups of from 8 to 20 carbon atoms and two or three small aliphatic (e.g. methyl) groups, for instance, cetyltrimethylammonium chloride. Further examples are given in well-known text books such as "Household Cleaning, Care and Maintenance Products", Edts. Herrmann G. Hauthal, G. Wagner, 1st Edition, Verlag für Chem. Industrie H. Ziolkowsky 2004, or "Kosmetische Emulsionen und Cremes", Gerd Kutz, Verlag für Chem. Industrie H. Ziolkowsky 2001.

The abrasive composition of the present invention may further comprise ingredients which aid in its cleaning performance. For example, the composition may contain detergent builders and mixtures of such builders in an amount of up to 25 wt.-%, based on the total amount of the composition. Suitable inorganic or organic detergent builders are known to the skilled person and may be selected, for example, from sodium tripolyphosphate or sodium aluminium silicate.

According to one embodiment, in addition to the components already mentioned, the abrasive cleaning composition further comprises dispersing agents, thickeners, preservatives, humectants, foaming agents, fluoride sources (e.g., in case the abrasive cleaning composition is an oral care product), flavouring agents, fragrances, colorants, active agents, and/or buffering systems. Examples of active agents are pharmaceutically, biologically or cosmetically active agents, biocides, or disinfecting agents. The abrasive cleaning composition of the present invention may also comprise further optional ingredients which are not mentioned here but known to skilled person.

According to one embodiment of the present invention, the abrasive cleaning composition is a cosmetic composition, preferably a cosmetic composition for topical application, and more preferably a facial cleanser, a body wash, a body scrub, a scrub shampoo, a massage cream, a body polisher, an exfoliator, a peeling cream, a food scrub, a microderm abrasion product, or an oral care composition. Examples of an oral care composition are a toothpaste, a toothpowder, a powder for dental powder blasting, or a chewable gum. According to one embodiment, the abrasive cleaning composition is a cosmetic composition having a pH value from 5.0 to 7.0, preferably from 5.5 to 6.5, and most preferably of about 6.

According to one embodiment, a cosmetic composition is provided comprising a surface-treated calcium carbonate with improved stability in environments with a pH of 4.5 to 7 is provided, wherein the cosmetic composition has a pH value from 5.0 to 7.0 and the surface-treated calcium carbonate is obtainable by a process comprising the steps of:
i) providing calcium carbonate,
ii) providing at least one acid having a pKₐ value from 0 to 8, when measured at 20°C,
iii) providing at least one conjugate base,
iv) contacting the calcium carbonate of step i), the at least one acid of step ii), and the at least one conjugate base of step iii) to form surface-treated calcium carbonate, and
v) drying the surface-treated calcium carbonate,
wherein the at least one acid of step ii) and/or the at least one conjugate base of step iii) are provided in form of an aqueous solution.

According to a further aspect of the present invention, the abrasive cleaning composition of the present invention is used for cleaning a surface.

According to a further aspect of the present invention, a method for cleaning a surface is provided, wherein the surface is contacted with an abrasive cleaning composition according to the present invention. The surface may be contacted with the inventive composition by applying the composition to the surface, for example, by spraying, pouring or squeezing. The abrasive cleaning composition may be applied to the surface by using an appropriate means such as a mop, a paper towel, a brush, or a cloth, soaked in the composition, which can be in neat or in diluted form.

According to one embodiment of the present invention, the surface is an inanimate surface, preferably selected from the group consisting of household hard surfaces, dish surfaces, leather surfaces, and automotive vehicle surfaces. Examples of household hard surfaces are working surfaces, ceramic surfaces, refrigerators, freezers, washing machines, automatic dryers, ovens, microwave ovens, ceramic cook tops, bathroom fittings or dishwashers. Examples of dish surfaces are dishes, cutlery, cutting boards, or pans.

According to another embodiment of the present invention, the surface is an animate surface, preferably selected from the group consisting of human skin, animal skin, human hair, animal hair, and tissues of the oral cavity such as teeth, gums, tongue or buccal surfaces.

According to one embodiment of the present invention, the method of the present invention further comprises a step of rinsing the composition.

According to a preferred embodiment, the abrasive cleaning composition of the present invention is used for cleaning an animate surface, and more preferably for cleaning an animate surface selected from the group consisting of human skin, animal skin, human hair, animal hair, and tissues of the oral cavity such as teeth, gums, tongue or buccal surfaces.

According to one embodiment, the surface to be cleaned is an inanimate surface, preferably selected from the group consisting of household hard surfaces, dish surfaces, leather surfaces, and automotive vehicle surfaces.

The scope and interest of the invention will be better understood based on the following examples which are intended to illustrate certain embodiments of the present invention and are non-limitative.

### Examples

### 1. Measurement Methods

In the following, measurement methods implemented in the examples are described.

### pH value

### a) Normal pH measurement

The pH of a suspension or solution was measured at 25°C using a Mettler Toledo Seven Easy pH meter and a Mettler Toledo InLab® Expert Pro pH electrode. A three point calibration (according to the segment method) of the instrument was first made using commercially available buffer solutions having pH values of 4, 7 and 10 at 20°C (from Sigma-Aldrich Corp., USA). The reported pH values are the endpoint values detected by the instrument (the endpoint was when the measured signal differed by less than 0.1 mV from the average over the last 6 seconds).

### b) pH measurement with regard to stability at pH 5 and 6

The pH of a suspension or solution was measured at 25°C using a Mettler Toledo DL58 Titrator and a Mettler Toledo DG 115-SC electrode. A three point calibration (according to the segment method) of the instrument was first made using commercially available buffer solutions having pH values of 4.01, 7.00 and 9.21 at 20°C (Duracal Buffers from Hamilton, Switzerland). The reported pH values are the endpoint values detected by the instrument (the endpoint was when the measured signal differed by less than 0.1 mV from the average over the last 2.5 seconds).

### BET specific surface area (SSA)

The BET specific surface area is measured via the BET process according to ISO 9277 using nitrogen, following conditioning of the sample by heating at 250°C for a period of 30 minutes. Prior to such measurements, the sample is filtered within a Büchner funnel, rinsed with deionised water and dried at 110°C in an oven for at least 12 hours.

### Particle size distribution

The weight median particle size of all particulate materials, e.g. natural ground or precipitated calcium carbonate, was determined by the sedimentation method, which is an analysis of sedimentation behaviour in a gravimetric field. The measurement was made with a Sedigraph™ 5100, Micromeritics Instrument Corporation. The method and the instrument are known to the skilled person and are commonly used to determine grain size of fillers and pigments in the range from 0.1 to 5 µm. The measurement was carried out in an aqueous solution of 0.1 wt.-% Na₄P₂O₇. The samples are dispersed using a high speed stirrer and supersonicated.

The volume determined median particle size *d*₅₀ of calcium carbonate and surface-treated calcium carbonate was evaluated using a Malvern Mastersizer 2000 Laser Diffraction System (Malvern Instruments Plc., Great Britain), using the Fraunhofer light scattering approximation. The method and instrument are known to the skilled person are commonly used to determine particle sizes of fillers and other particulate materials of > 5 µm. Although it is preferable to use the Sedigraph method for particle sizes of less than 5 µm it is also possible to do volume based particles size determination using the Malvern Mastersizer for particle sizes as low as 1 µm. The samples were dispersed using a high speed stirrer and in the presence of supersonics.

### 2. Materials

For the present invention the following buffer solutions were tested:

It has to be noted that for the preparation of each buffer solution deionized water was used.

### A: Phosphate buffer

999.2 g of a NaH₂PO₄ solution (0.1 mol/kg) and 20.8 g Na₂HPO₄ solution (0.1 mol/kg) were mixed. The final pH of this buffer was 5.0 and the total phosphate ionic concentration was 0.1 mol/kg.

### B: Phosphate citrate buffer

257.3 g Na₂HPO₄ solution (0.1 mol/kg), 269.1 g citric acid solution (0.1 mol/kg) and 492.7 g deionized water were mixed. The final pH of this buffer was 5.0, the total phosphate ionic concentration was 0.05 mol/kg, and the total citrate ionic concentration was 0.026 mol/kg.

### C: Citrate buffer

A first solution was prepared by placing 1 000 g sodium dihydrogen citrate solution (0.1 mol/kg) in a beaker, and adding 48.2 g trisodium citrate to the beaker while stirring with a magnetic stirring bar. The total citrate concentration of said first solution was 0.27 mol/kg.

365 g of said first solution and 635 g deionized water were combined and mixed. Then a 0.1 mol/kg citric acid solution was added until a final pH of 5.0 was reached. The final buffer solution had a total citrate ion concentration of 0.1 mol/kg.

The following chemicals were used to prepare the above solutions: Sodium dihydrogen phosphate monohydrate (NaH₂PO₄.H₂O, Merck KGaG), di-sodium hydrogen phosphate di-hydrate (Na₂HPO₄.2H₂O, Merck KGaA), citric acid anhydrous (H₃C₆H₅O₇, Sigma Aldrich), sodium di-hydrogen citrate anhydrous (NaH₂C₆H₅O₇, Sigma Aldrich) and tri-sodium citrate di-hydrate (Na₃C₆H₅O₇.2H₂O, Sigma Aldrich).

### Calcium carbonate

The calcium carbonate used was a natural ground calcium carbonate from Karabiga, Turkey, having a *d*₁₀ (volume determined) of 200 µm, a *d*₂₀ (volume determined) of 243 µm, a *d*₅₀ (volume determined) of 362 µm, a *d*₉₀ (volume determined) of 640 µm, and a *d*₉₈ (volume determined) of 811 µm), and a BET specific surface area less than 1.0.

### 3. Examples

### Example 1 - Preparation of surface-treated calcium carbonate

Three samples of surface-treated calcium carbonate were prepared by treating the calcium carbonate with any one of the three prepared buffer solutions as follows:

1 000 g buffer solution was added to a beaker. Under stirring 550 g calcium carbonate was added and the mixture was stirred for 5 minutes.

After letting the mixture sit for 20 to 24 hours, the pH of the mixture was measured. The measured pH values are given in Table 1 below. Then, the mixture was filtered through a 100 µm sieve. The obtained residue on the sieve was washed with approximately 500 g of tap water in a beaker for about half a minute and the mixture was filtered again. Then the residue obtained on the sieve was dried in an oven at 90°C over night.

**Table 1: pH values of solution after preparation of surface-treated calcium carbonate.**

| Sample | Buffer solution | pH value | BET |
|---|---|---|---|
| | | | [m²/g] |
| 1 | A | 6.8 | 1.5 |
| 2 | B | 6.8 | < 1.0 |
| 3 | C | 8.0 | < 1.0 |

### Example 2 - Stability of surface-treated calcium carbonate

The samples prepared according to Example 1 were analyzed with regard to their stability under acidic conditions at pH 5 and pH 6 by titrating the samples with acetic acid (2.0 mol/l). The untreated calcium carbonate was also analyzed as comparative sample.

### Stability at pH 5

A 2.0 mol/l solution of acetic acid was prepared from concentrated acetic acid (from Sigma-Aldrich, CAS No. 64-19-7, puriss. 99-100%).

0.9 to 1.1 g of the sample was weighted into a plastic cup, and 100 ml of an ethanol (A15-A ethanol absolutus from Alcosuisse) - water mixture (60/40 v/v) was added.

The titration of the sample was performed in triplicate with the prepared acetic acid solution over time in order to reach a pH value of 5.0 with a control band of 0.5. The titration was performed over a time period of 60 minutes.

### Stability at pH 6

A 0.1 mol/l solution of acetic acid was prepared from concentrated acetic acid (from Sigma-Aldrich, CAS No. 64-19-7, puriss. 99-100%).

Exactly 1.2 g of the sample was weighted in a plastic cup (accuracy 0.1 mg), and 100 ml of an ethanol (A15-A ethanol absolutus from Alcosuisse) - water mixture (60/40 v/v) was added.

The titration of the sample was performed in triplicate with the prepared acetic acid solution over time in order to reach a pH value of 6.0 with a control band of 0.1. The titration was performed over a time period of 60 minutes.

The final results are listed in the following Table 2 (results at pH 5) and Table 3 (results at pH 6). The results given in Tables 2 and 3 show the acid consumption per hour, measured between 15 and 60 minutes.

**Table 2: Acid stability at pH 5.**

| Sample | Acid consumption per hour [mmol acetic acid / kg CaCO₃] | Reduction of acid consumption per hour [%] [(aₙ/b)* 100] with n = 1,2,3 |
|---|---|---|
| Calcium carbonate (comparative) (b) | 10 605 ± 223 | |
| Sample 1 (a₁) | 2 861 ±168 | 73 |
| Sample 2 (a₂) | 1 973 ±41 | 81 |
| Sample 3 (a₃) | 2 232 ±109 | 79 |

**Table 3: Acid stability at pH 6.**

| Sample | Acid consumption per hour [mmol acetic acid / kg CaCO₃] | Reduction of acid consumption per hour [%] [(aₙ/b)* 100] with n = 1,2,3 |
|---|---|---|
| Calcium carbonate (comparative) (b) | 399 ± 17 | |
| Sample 1 (a₁) | 124 ± 5 | 69 |
| Sample 2 (a₂) | 27 ± 2 | 93 |
| Sample 3 (a₃) | 35 ± 6 | 91 |

As can be seen from the above results, both at pH 5 and pH 6, the inventive surface-treated calcium carbonate samples 1 to 3 showed less acid consumption per hour, and, thus, an improved acid stability compared to the comparative untreated calcium carbonate sample.

### Example 3 - Preparation of body scrub formulations

Each of the different body scrub formulations was prepared according to the following guide formulation:

| | | **wt.- %** | **grams** |
|---|---|---|---|
| **A**) | Tegin | 6.00 | 18.00 |
| | Isopropyl Myristate | 2.00 | 6.00 |
| | Phenonip | 1.00 | 3.00 |
| | Lanette O | 3.00 | 9.00 |
| | | | |
| **B)** | Water | 42.50 | 127.50 |
| | Panthenol 75L | 1.00 | 3.00 |
| | Akucel 3285 | 0.50 | 1.50 |
| | | | |
| **C)** | Marlinat 242/70 | 20.00 | 60.00 |
| | EUR-AMID N2 | 2.00 | 6.00 |
| | Dow Corning HMW 2220 non-ionic Emulsion | 1.00 | 3.00 |
| | EURONAC AMF ULTRA | 1.00 | 3.00 |
| | | | |
| **D**) | Abrasive material | 20.00 | 60.00 |
| | | 100.00 | **300.00** |

The abrasive material used in part D is listed in Table 4 below.

**Table 4: Composition of prepared body scrub formulations.**

| Formulation | wt.-% abrasive material, based on total weight of formulation | Abrasive material |
|---|---|---|
| 1 (comparative) | 20 | calcium carbonate |
| 2 | 20 | sample 1 |
| 3 | 20 | sample 2 |
| 4 | 20 | sample 3 |

### Procedure:

1. Melt part A and B separately and keep warm at 85°C (in a water bath)
2. Mix both parts (A and B) under intensive agitation using a Heidolph RZR 2041 mixer at 800 - 1 000 rpm.
3. Homogenize intensively by using a ultra Turrax™ (from IKA, Staufen, Germany) and cool down to room temperature under agitation using a Heidolph RZR 2041 mixer (300 to 350 rpm).
4. Add part C step by step and mix under slow agitation using Heidolph RZR 2041 mixer (150 to 200 rpm) until part C is homogenously incorporated (appr. 10 - 15 minutes).
5. Add part D step by step and mix under slow agitation using Heidolph RZR 2041 mixer (150 to 200 rpm; appr. 10 - 15 minutes) until a homogenous mixture is obtained. The final product can be filled in glass flasks.

**Table 5 below is an overview over the type of product used in the above guide formulation as well as the suppliers of these compounds.**

| **Ingredients** | **INCI Nomenclature** | **Suppliers** |
|---|---|---|
| Tegin | Glyceryl Stearate SE | 1) |
| Isopropyl myristate | Isopropyl Myristate | 2) |
| Phenochem | Phenoxyethanol (and) Methylparaben (and) Ethylparaben (and) Butylparaben (and) Propylparaben (and) Isobutylparaben | 3) |
| Lanette O | Cetearyl Alcohol | 4) |
| Water dem. | Aqua (Water) | |
| D-Panthenol 75 L | Panthenol | 5) |
| Akucell AF 3285 | Cellulose Gum | 6) |
| Marlinat 242/70 | Sodium Laureth Sulfate | 7) |
| Euro-Amid N2 | Cocamide MIPA (and) Laureth-4 | 8) |
| Dow Corning HWM | Divenyldimethicone/Dimethicone | 9) |
| 2220 non-ionic emulsion | Copolymer (and) C12-C13 Pareth -3 (and) C12-C13 Pareth-23 | |
| Euronac AMF Ultra | Glycol Distearate (and) Cocamidopropyl Betaine | 8) |
| Omyacare^{®} S 20-KA | Calcium Carbonate | 10) |

### Suppliers:

| | | | |
|---|---|---|---|
| **1** | Hänseler AG, Switzerland | **2** | Omya Hamburg GmbH, Germany |
| **3** | SLI Chemicals GmbH, Germany | **4** | Cognis GmbH, Germany |
| **5** | DSM, Switzerland | **6** | Akzo Nobel Functional Chemicals BV; Netherlands |
| **7** | Sasol, Germany | **8** | EOC Surfactants, Belgium |
| **9** | Dow Corning Corporation, USA | **10** | Omya AG, Switzerland |
| **11** | Clariant (Schweiz) AG, Switzerland | | |

### Example 4 - Sensory evaluation of the prepared body scrub formulations

The sensory properties of the prepared body scrub formulations 1 to 4 were tested by applying approx. 0.075 ml of the body scrub on the hand or the finger by using a small spoon. The sensory properties of the formulations mentioned below were marked with a rating from 0 to 10, wherein 0 means "not good" and 10 means "very good".

### Evalatated sensory properties:

### - Aspect

| Descriptor | Description |
|---|---|
| Separation | It is a visible separation between the both phases. |
| No separation | No separation is visible between the both phases. |
| Shiny | The product reflects the light under a standard lamp. |
| No shiny | The product is mat and reflects no light under a standard lamp. |
| Reaction | Presence of a reaction between the exfoliator and the emulsion. |
| No reaction | No reaction is visible between the exfoliator and the emulsion. |

### - Hand movement

| Descriptor | Description |
|---|---|
| Fluid | When the product is placed between the thumb and index finger by applying a pressure, it has no holding, flows off the fingers and moves away from the pressure zone. No resistance is felt. |
| Non fluid | When the product is placed between the thumb and index finger by applying a pressure, there is no flow between the both fingers and it moves not away from the pressure zone. A resistance is felt. |
| Stringy | When the product is placed between the thumb and index finger and a distance is slowly applied between the both fingers, it is a continuous string. It breaks when the distance between the fingers becomes too large. |
| Non stringy | When the product is placed between the thumb and index finger and a distance is slowly applied between the both fingers, there is no string/elongated filament formed. |
| Slippery | When the product is placed between the thumb and index finger and a movement of friction is made, there is no resistance of particles. The particles make the movement easier. |
| Non slippery | When the product is placed between the thumb and index finger and a movement of friction is made, there is a resistance. The particles restrict the movement. |

### - Spread

| Descriptor | Description |
|---|---|
| Spread | Between 5 and 10 turns on the hand, there is a good distribution of product. |
| Non spread | Between 5 and 10 turns on the hand, there is no repartition of product. |
| Foaming | Between 10 and 15 rotations on the hand, the product forms foam. |
| Non foaming | No foaming is visible between 10 and 15 rotations on the hand. |
| Non Soft (exfoliation) | The particles give no pleasant sensation during the application on the skin. |
| Soft (exfoliation) | A pleasant and soft exfoliation is felt during the application. |

### - After one minute

| Descriptor | Description |
|---|---|
| Greasy | When the product is placed between the thumb and index finger and a movement of friction is applied, the products have an oily aspect. |
| Non greasy | When the product is placed between the thumb and index finger and a movement of friction is applied, there is no oily aspect. |
| Sticky | By performing a pressure with index finger on the hand, there is an adhesion. |
| Non sticky | By performing a pressure with index finger on the hand, there is no adhesion. |

### - After two minutes

| Descriptor | Description |
|---|---|
| Penetrating | The product is disappearing and no residue is detected when touching skin after two minutes. |
| Non penetrating | There can still a residue be detected on the skin after touching the skin after two minutes. |

### - During cleaning

| Descriptor | Description |
|---|---|
| Greasy | Under water, when the product is placed between the thumb and index finger and a movement of friction is applied, the products give oily aspect. |
| Non greasy | Under water, when the product is placed between the thumb and index finger and a movement of friction is applied, there is no oily aspect. |
| Slippery | Under water, by performing a pressure with index finger on the hand, there is no resistance and good sliding. |
| Non slippery | Under water, by performing a pressure with index finger on the hand, there is a resistance and no sliding on the hand. |

### - After rinsing and drying

| Descriptor | Description |
|---|---|
| Greasy | After one minute, when the skin is drying, an oily aspect is felt when a movement is made between the thumb and index fingers. |
| Non greasy | After one minute, when the skin is drying, no oily aspect is felt when a movement is made between the thumb and index fingers. |
| Soft | After one minute, when the index finger makes a sliding on the hand, the skin is soft and a dry touch is felt. |
| Non soft | After one minute, when the index finger makes a sliding on the hand, the skin is rough and no soft touch is felt. |

### Results:

**Table 5: Sensory properties after 6 weeks storage at room temperature.**

| Formulation | 1 | 2 | 3 | 4 |
|---|---|---|---|---|
| Aspect | | | | |
| Shiny | 8 | 8 | 8 | 7 |
| Reaction | 0 | 0 | 0 | 0 |

| Hand movement | | | | |
|---|---|---|---|---|
| Fluid | 4 | 5 | 5 | 4 |
| Stringy | 7 | 6 | 2 | 3 |
| Slippery | 5 | 7 | 4 | 4 |

| Spread | | | | |
|---|---|---|---|---|
| Foaming | 5 | 6 | 6 | 6 |
| Spread | 4 | 7 | 6 | 6 |
| Soft (exfoliation) | 2 | 3 | 3 | 3 |

| After one minute | | | | |
|---|---|---|---|---|
| Greasy | 4 | 6 | 4 | 3 |
| Sticky | 5 | 6 | 4 | 5 |

| After two minutes | | | | |
|---|---|---|---|---|
| Penetrating | 8 | 8 | 8 | 8 |

| During cleaning | | | | |
|---|---|---|---|---|
| Greasy | 6 | 6 | 6 | 6 |
| Slippery | 7 | 8 | 8 | 7 |

| After rinsing and drying | | | | |
|---|---|---|---|---|
| Soft | 8 | 9 | 9 | 8 |
| Greasy | 5 | 4 | 4 | 4 |

**Table 6: Sensory properties after 6 weeks storage at 5°C.**

| Formulation | 1 | 2 | 3 | 4 |
|---|---|---|---|---|
| Aspect | | | | |
| Shiny | 8 | 8 | 8 | 8 |
| Reaction | 0 | 0 | 0 | 0 |

| Hand movement | | | | |
|---|---|---|---|---|
| Fluid | 4 | 5 | 4 | 6 |
| Stringy | 3 | 1 | 2 | 4 |
| Slippery | 7 | 8 | 4 | 6 |

| Spread | | | | |
|---|---|---|---|---|
| Foaming | 6 | 6 | 6 | 6 |
| Spread | 4 | 7 | 5 | 5 |
| Soft (exfoliation) | 2 | 2 | 2 | 2 |

| After one minute | | | | |
|---|---|---|---|---|
| Greasy | 2 | 3 | 3 | 2 |
| Sticky | 2 | 2 | 3 | 2 |

| After two minutes | | | | |
|---|---|---|---|---|
| Penetrating | 8 | 8 | 8 | 8 |

| During cleaning | | | | |
|---|---|---|---|---|
| Greasy | 6 | 7 | 7 | 7 |
| Slippery | 7 | 8 | 8 | 7 |

| After rinsing and drying | | | | |
|---|---|---|---|---|
| Soft | 8 | 8 | 8 | 8 |
| Greasy | 5 | 2 | 3 | 4 |

**Table 7: Sensory properties after 6 weeks storage at 40°C.**

| Formulation | 1 | 2 | 3 | 4 |
|---|---|---|---|---|
| Aspect | | | | |
| Shiny | 7 | 8 | 8 | 8 |
| Reaction | 0 | 0 | 0 | 0 |

| Hand movement | | | | |
|---|---|---|---|---|
| Fluid | 7 | 6 | 7 | 7 |
| Stringy | 3 | 4 | 3 | 3 |
| Slippery | 7 | 6 | 6 | 7 |

| Spread | | | | |
|---|---|---|---|---|
| Foaming | 6 | 7 | 7 | 6 |
| Spread | 7 | 6 | 7 | 6 |
| Soft (exfoliation) | 4 | 4 | 4 | 3 |

| After one minute | | | | |
|---|---|---|---|---|
| Greasy | 3 | 2 | 2 | 3 |
| Sticky | 2 | 4 | 2 | 2 |

| After two minutes | | | | |
|---|---|---|---|---|
| Penetrating | 8 | 8 | 8 | 7 |

| During cleaning | | | | |
|---|---|---|---|---|
| Greasy | 6 | 7 | 6 | 6 |
| Slippery | 8 | 8 | 8 | 7 |

| After rinsing and drying | | | | |
|---|---|---|---|---|
| Soft | 8 | 8 | 8 | 8 |
| Greasy | 6 | 2 | 2 | 2 |

As can be gathered from the results compiled in Tables 5 to 7 above, compared to the others formulation, inventive formulation 2 showed the best "slippery" effect on the skin. Foaming is similar for all, but better at 5°C and 40°C. Spread is improved for the samples stored at 40°C, because the viscosity decreased at the higher storage temperature. The best spread remains the same for the formulations 2 and 4.

After rinsing and cleaning of the skin, the evaluation of the softness (descriptor "soft") gave practically the same results for all formulae. A greasy aspect leads to a noticeable difference between the samples under peeling.

In conclusion, all inventive body scrub formulations showed good sensory performance, confirming that the surface-treated calcium carbonate obtained by the process of the present invention is suitable for cosmetic products such as body scrub.

## Claims

1. A process for producing a surface-treated calcium carbonate with improved stability in environments with a pH of 4.5 to 7, wherein the process comprises the steps of:
i) providing calcium carbonate,
ii) providing at least one acid having a pKₐ value from 0 to 8, when measured at 20°C,
iii) providing at least one conjugate base,
iv) contacting the calcium carbonate of step i), the at least one acid of step ii), and the at least one conjugate base of step iii) to form surface-treated calcium carbonate,
wherein the at least one acid of step ii) and/or the at least one conjugate base of step iii) are provided in form of an aqueous solution.

2. The process of claim 1, wherein step iv) comprises the steps of:
a1) contacting the calcium carbonate of step i) and the at least one acid of step ii) to form a pre-treated calcium carbonate, and
a2) contacting the pre-treated calcium carbonate of step a1) with the at least one conjugate base of step iii) to form surface-treated calcium carbonate,
wherein at least the at least one acid of step ii) is provided in form of an aqueous solution.

3. The process of claim 1, wherein step iv) comprises the steps of:
b1) contacting the calcium carbonate of step i) and the at least one conjugate base of step iii) to form a pre-treated calcium carbonate, and
b2) contacting the pre-treated calcium carbonate of step b1) with the at least one acid of step ii) to form surface-treated calcium carbonate,
wherein at least the at least one conjugate base of step iii) is provided in form of an aqueous solution.

4. The process of claim 1, wherein the at least one acid of step ii) and the at least one conjugate base of step iii) are provided together in form of an aqueous buffer solution, and in step iv) the calcium carbonate of step i) is contacted with the aqueous buffer solution to form surface-treated calcium carbonate.

5. The process of claim 4, wherein step iv) comprises the steps of:
c1) mixing the calcium carbonate of step i), the aqueous buffer solution, and optionally water, to form an aqueous suspension of surface-treated calcium carbonate, and
c2) separating the surface-treated calcium carbonate from the aqueous suspension obtained from step c1).

6. The process of any one of the preceding claims, wherein the calcium carbonate of step i) is natural ground calcium carbonate, precipitated calcium carbonate, dolomite, agglomerates or aggregates thereof, or a mixture of the aforementioned materials, and preferably natural ground calcium carbonate.

7. The process of any one of the preceding claims, wherein the calcium carbonate of step i) is in form of particles having a volume determined median particle size *d*₅₀ from 1 to 1 500 µm, preferably from 25 to 1 400 µm, more preferably from 100 from 1 200 µm, even more preferably from 200 to 1 000 µm, and most preferably from 300 to 800 µm.

8. The process of any one of the preceding claims, wherein the at least one acid of step ii) is selected from the group consisting of phosphoric acid, citric acid, sulphurous acid, boric acid, acetic acid, tartaric acid, formic acid, propanoic acid, oxalic acid, phosphonic acid, their acidic salts, and mixtures thereof, preferably selected from the group consisting of phosphoric acid, citric acid, tartaric acid, oxalic acid, their acidic salts, and mixtures thereof, and most preferably selected from the group consisting of phosphoric acid, citric acid, their acidic salts, and mixtures thereof.

9. The process of any one of the preceding claims, wherein the at least one conjugate base of step iii) is an alkali metal salt and/or alkaline earth metal salt of an acid having a pKₐ value from 0 to 8, when measured at 20°C, preferably the at least one conjugate base of step iii) is an alkali metal salt and/or alkaline earth metal salt of an acid selected from the group consisting of phosphoric acid, citric acid, sulphurous acid, boric acid, acetic acid, tartaric acid, formic acid, propanoic acid, oxalic acid, phosphonic acid, and mixtures thereof, more preferably the at least one conjugate base of step iii) is a sodium and/or potassium salt of an acid selected from the group consisting of phosphoric acid, citric acid, sulphurous acid, boric acid, acetic acid, tartaric acid, formic acid, propanoic acid, oxalic acid, phosphonic acid, and mixtures thereof, and most preferably the at least one conjugate base of step iii) is a sodium and/or potassium salt of an acid selected from the group consisting of phosphoric acid, citric acid, and mixtures thereof.

10. The process of any one of claims 4 to 9, wherein the aqueous buffer solution has a pH value from 4.0 to 7.5, preferably from 4.5 to 6.0, and most preferably from 4.8 to 5.5.

11. The process of any one of claims 4 to 10, wherein the aqueous buffer solution has a concentration from 0.01 mol/kg to 2 mol/kg, preferably from 0.04 to 1.0 mol/kg, more preferably from 0.06 to 0.5 mol/kg, even more preferably from 0.07 to 0.4 mol/kg, and most preferably from 0.08 to 0.2 mol/kg.

12. The process of any one of the preceding claims, wherein the calcium carbonate of step i) is in form of particles having a specific BET surface area in the range of > 0 to 5 m²/g, preferably in the range of > 0 to 2 m²/g, and more preferably in the range of > 0 to 1 m²/g.

13. The process of any one of claims 1 to 12, further comprising a step v) of drying the surface-treated calcium carbonate.

14. A suspension of a surface-treated calcium carbonate with improved stability in environments with a pH of 4.5 to 7 obtainable by a process according to any one of claims 1 to 12.

15. The suspension of a surface-treated calcium carbonate of claim 14, wherein the surface-treated calcium carbonate has a reduced acid consumption per hour at pH 5 and pH 6 when treated with acetic acid, the reduction being at least 25 %, more preferably at least 50 %, and most preferably at least 70 %.

16. The suspension of a surface-treated calcium carbonate of claim 14 or 15, wherein the surface-treated calcium carbonate is in form of particles having a specific BET surface area in the range of > 0 to 5 m²/g, preferably in the range of > 0 to 2 m²/g, and more preferably in the range of > 0 to 1.6 m²/g.

17. A dried surface-treated calcium carbonate with improved stability in environments with a pH of 4.5 to 7 obtainable by a process according claim 13.

18. The dried surface-treated calcium carbonate of claim 17, wherein the surface-treated calcium carbonate has a reduced acid consumption per hour at pH 5 and pH 6 when treated with acetic acid, the reduction being at least 25 %, more preferably at least 50 %, and most preferably at least 70 %.

19. The dried surface-treated calcium carbonate of claim 17 or 18, wherein the surface-treated calcium carbonate is in form of particles having a specific BET surface area in the range of > 0 to 5 m²/g, preferably in the range of > 0 to 2 m²/g, and more preferably in the range of > 0 to 1.6 m²/g.

20. An abrasive cleaning composition comprising a suspension of a surface-treated calcium carbonate according to claims 14 to 16 or a dried surface-treated calcium carbonate according to claims 17 to 19.

21. Use of a suspension of surface-treated calcium carbonate according to claims 14 to 16 as abrasive material, preferably as abrasive material for cleaning application.

22. Use of a dried surface-treated calcium carbonate according to claims 17 to 19 as abrasive material, preferably as abrasive material for cosmetic application and/or cleaning application, more preferably as abrasive material for cosmetic application, and most preferably as abrasive material for topical skin application.

23. Use of an abrasive cleaning composition according to claim 20 for cleaning a surface, preferably for cleaning an animate surface, and more preferably for cleaning an animate surface selected from the group consisting of human skin, animal skin, human hair, animal hair, and tissues of the oral cavity such as teeth, gums, tongue or buccal surfaces.
